# EUROPEAN PATENT SPECIFICATION

(11) **EP 2 163 645 B1**
(45) Date of publication and mention of the grant of the patent: **26.09.2012**
(21) Application number: 08711525.9
(22) Date of filing: 19.02.2008
(51) Int. Cl.: G01N 33/68

(54) **METHOD OF DETECTING CEREBRAL STROKE OR ASYMPTOMATIC CEREBRAL INFARCTION USING ACROLEIN, INTERLEUKIN-6 AND CRP CONTENTS, POLYAMINE OXIDASE ACTIVITY OR POLYAMINE OXIDASE PROTEIN CONTENT AND SUBJECT'S AGE AS INDICATIONS**
VERFAHREN ZUM NACHWEIS EINES GEHIRNSCHLAGS ODER ASYMPTOMATISCHEN GEHIRNINFARKTS UNTER VERWENDUNG VON ACROLEIN-, INTERLEUKIN-6- UND CRP-GEHALTEN, POLYAMINOXIDASE-AKTIVITÄT ODER POLYAMINOXIDASE-PROTEINGEHALT SOWIE DES PATIENTENALTERS ALS INDIKATIONEN
PROCÉDÉ DE DÉTECTION D'UN ACCIDENT VASCULAIRE CÉRÉBRAL OU D'UN INFARCTUS CÉRÉBRAL ASYMPTOMATIQUE À L'AIDE DE TENEURS EN ACROLÉINE, INTERLEUKINE-6 ET CRP, ACTIVITÉ POLYAMINE OXYDASE OU TENEUR EN PROTÉINE POLYAMINE OXYDASE ET DE L'ÂGE DU SUJET COMME INDICAT

(30) Priority: 18.05.2007 JP 2007132239
(43) Date of publication of application: 17.03.2010
(73) Proprietor: National University Corporation Chiba University, Chiba-shi Chiba 263-8522 (JP)
(72) Inventor: IGARASHI, Kazuei, Chiba-shi Chiba 260-8675 (JP); NAMIKI, Shinjyuro, Tokyo 150-0012 (JP); YOSHIDA, Madoka, Chiba-shi Chiba 260-8675 (JP)
(74) Representative: Webber, Philip Michael
(86) International application number: PCT/JP2008/052701
(87) International publication number: WO 2008/142888

(56) References cited:
- EP-A1- 1 729 129
- JP-A- 05 176 932
- JP-A- 2005 304 476
- CARROLL MARY F ET AL: "Timing of antioxidant vitamin ingestion alters postprandial proatherogenic serum markers." CIRCULATION, vol. 108, no. 1, 8 July 2003 (2003-07-08), pages 24-31, XP002578972 ISSN: 0009-7322
- BERNARD D ET AL: "GAMMA AMINO-ALPHA-ACETYLENIC EPOXIDES PREPARATION AND BIOLOGICAL ACTIVITY DUE TO AN ALDEHYDE REDUCTASE INHIBITION" TETRAHEDRON, vol. 45, no. 5, 1989, pages 1429-1440, XP002578973 ISSN: 0040-4020
- TOMITORI HIDEYUKI ET AL: "Polyamine oxidase and acrolein as novel biochemical markers for diagnosis of cerebral stroke" STROKE, LIPPINCOTT WILLIAMS & WILKINS, US LNKD- DOI:10.1161/01.STR.0000190004.36793.2D, vol. 36, no. 12, 1 December 2005 (2005-12-01), pages 2609-2613, XP009121762 ISSN: 0039-2499
- HOSHI T ET AL: "Relations of serum high-sensitivity C-reactive protein and interleukin-6 levels with silent brain infarction" STROKE 200504 US LNKD- DOI:10.1161/01.STR.0000158915.28329.51, vol. 36, no. 4, April 2005 (2005-04), pages 768-772, XP002578974 ISSN: 0039-2499
- SILVESTRI ANTONELLO ET AL: "Plasma levels of inflammatory C-reactive protein and interleukin-6 predict outcome in elderly patients with stroke." JOURNAL OF THE AMERICAN GERIATRICS SOCIETY SEP 2004 LNKD- PUBMED:15341576, vol. 52, no. 9, September 2004 (2004-09), pages 1586-1587, XP002578975 ISSN: 0002-8614
- KATAGIRI D. ET AL.: 'Nokosoku Biomarker no Kaihatsu' PRE SYMPTOMATIC MEDICINE AND ANTI AGING vol. 16, no. 1, 27 April 2007, pages 31 - 35
- HOSHI T. ET AL.: 'Mushokosei Nokosoku to Ensho Hanno no Kanren' THE JOURNAL OF ADULT DISEASES vol. 35, no. 5, 2005, page 552
- SATO M. ET AL.: 'Manseiki Nosotchu Kanja ni Okeru Kokando CRP, Interleukin 6 no Josho' JAPANESE JOURNAL OF STROKE vol. 26, no. 3, 2004, pages 423 - 429
- NISHIMURA H. ET AL.: 'Nosotchu Hassho.Saihatsu no Yobo - Seikatsu Shukan no Kaizen wa Yobo ni Yakudatsuka Seikatsu Shukan to Nosotchu Sonoto no Kiken Inshi tono Sogo Kankei' THE JOURNAL OF ADULT DISEASES vol. 35, no. 4, 2005, pages 407 - 414
- YAMAMOTO H. ET AL.: 'Shinbo Saido ni Okeru Mushokosei Nokosoku no Kikensei ni Taisuru, Shinkei Taiekisei Inshi ni Kansuru Kento' DAI 58 KAI KOKURITSU BYOIN RYOYOJO SOGO IGAKUKAI KOEN SHOROKUSHU 06 October 2003, page 396 + ABSTR. NO. P3-H-B12-7
- REBROVA O.Y. ET AL.: 'Development and realization of the artificial neural network for diagnostics of stroke type' LECTURE NOTES IN COMPUTER SCIENCE vol. 3696, 2005, pages 659 - 663, XP019018106
- BRANES R.W. ET AL.: 'Neural networks for ischemic stroke' JOURNAL OF STROKE & CEREBROVASCULAR DISEASES vol. 15, no. 5, 2006, pages 223 - 227, XP005643527
- OTSUKA T.: 'Nosotchu Yogo Yosoku ni Okeru Tobu CT Shoken no Yuyosei ni Tsuite' JAPANESE JOURNAL OF REHABILITATION MEDICINE vol. 40, no. 7, 2003, pages 443 - 452

## Description

### Technical Field

The present invention relates to methods of detecting cerebral stroke or asymptomatic cerebral infarction using the contents of acrolein, and interleukin-6 and CRP which are inflammatory markers, polyamine oxidase activity or polyamine oxidase protein contents and subjects' ages as indications.

### Background Art

In Japan, the number of deaths caused by cerebrovascular diseases is the third largest after those by neoplasm malignants and cardiac diseases. Sequela following the affection of the cerebrovascular diseases not only pose serious problems for patients in the daily life of their own, e.g., involving paralysis or akinesia, but also contribute to increased psychic stress on caregivers. Cerebral stroke which is the majority of the cerebrovascular diseases is a disease difficult to early detect and early treat. Although initiation of treatment of patients in the stages of cerebral infarctions without any subjective symptoms of cerebral infarctions, such as hemiplegia, hemiparesis, numbness, decreased sensation, hand-foot movement disorders, disturbance of consciousness and language disorders, (asymptomatic cerebral infarctions) is effective, the majority of cases with asymptomatic cerebral infarctions are accidentally found using diagnostic imaging and there are no diagnosis marker for utilized in, e.g., blood examination or urinalysis under present circumstances. Therefore, development of simple and highly-reliable diagnosis methods which are not methods requiring expensive devices, such as diagnostic imaging, is desired.

Acrolein and polyamine oxidases generating it from polyamine have been known as biomarkers having correlations with cerebral infarction. Acrolein is detoxicated by aldehyde dehydrogenase in a cell but, when leaked extracellularly, demonstrates a strong toxicity. Therefore, acrolein is considered to have a high correlation with a cell injury score, and thus its correlations with symptoms such as renal disorders, cerebral stroke and asymptomatic cerebral infarction have been studies. See non-patent documents 1 to 3 and patent documents 1 and 2.

### Non-Patent Document 1

Sakata, K. et al (2003) Biochem. Biophys. Res. Commun. 305, 143-149 Non-Patent Document 2
Tomitori, H. et al (2005) Stroke 36, 2609-2613 Non-Patent Document 3
Igarashi, K. et al (2006) Amino Acids 31, 477-483 Patent Document 1
Japanese Unexamined Patent Application Publication No. 2002-95942 Patent Document 2
Japanese Unexamined Patent Application Publication No. 2005-304476 its corresponding European Patent publication is EP1 729 129 A1.

However, for example, the accuracy of conventional diagnosis and screening methods for cerebral stroke and asymptomatic cerebral infarction based on acrolein or polyamine oxidase activity as described in the patent document 2 was still not sufficient, particularly in terms of practical use.

In contrast, interleukin-6, which was found as a B cell differentiation-inducing factor, is known to be a malignant cell growth factor for multiple myeloma and to be involved in various inflammatory diseases and autoimmune diseases. Therefore, studies of interleukin-6 as a biomarker for cerebral stroke have been conducted. See non-patent documents 4 and 5 and a patent document 3.

CRP (C-reactive protein) was also found as a serum protein (β-globulin) which undergoes a precipitation reaction with C-polysaccharide extracted from a cell wall in *Streptococcus pneumoniae*, and blood CRP concentrations are known to be increased in many diseases such as infections (particularly, bacterial infections), myocardial infarction and autoimmune diseases. In recent years, technological improvement in measuring devices has leaded to development of methods for measuring high sensitivity CRP (hs-CRP), which allow sensing of even extremely mild inflammatory reactions without definite inflammatory diseases such as infections and malignant tumors (minimum detectable sensitivity: 0.01 mg/L). In a large clinical study by Ridker et al., CRP was reported to become an independent prediction marker for ischemic heart disease. Therefore, studies of CRP as a biomarker for cerebral stroke have been conducted. See non-patent documents 6, 7 and 8 and a patent document 3.
Non-Patent Document 4
   Smith, C. J. et al (2004) BMC Neurol. 15, 4:2
Non-Patent Document 5
   Tzoulaki, I. et al (2006) Circulation. 115, 2119-2127
Non-Patent Document 6
   Ridker PM. et al (2002) Engl J Med 347, 1557-1565
Non-Patent Document 7
   Wakugawa, Y. et al (2006) Stroke 37, 27-32
Non-Patent Document 8
   Tzoulaki, I. et al (2006) Circulation. 115, 2119-2127
Patent Document 3
   Japanese Unexamined Patent Application Publication No. 2005-522669

However, there are no cases of using interleukin-6 or CRP individually as a biomarker for diagnosis of cerebral infarction, and a technique of distinguishing the stroke type of a patient after the onset of a cerebral stroke symptom in combination of interleukin-6 or CRP with B-type natriuretic peptide, matrix metalloprotease-9, S-100β, thrombin-antithrombin III complex and one or more types of von Willebrand factors is disclosed. Furthermore, neither suggestion nor teaching about the relationships of, in particular, samples from patients with asymptomatic cerebral infarction before the onset of stroke symptoms, with interleukin-6 and CRP are given.

Carroll and Schade, *Circulation* **108**, 24-31 (2003) shows measurement of C-reactive protein, interleukin 6 and malonyldialdehyde in blood samples of test subjects. Hoshi et al., Stroke 36, 768-772 (2005) shows increased levels of C-reactive protein and interleukin 6 in serum of patients with silent brain infarctions.

### Disclosure of the Invention

### Problems to be Solved by the Invention

Therefore, an object of the invention is to provide a method of detecting or screening cerebral stroke or asymptomatic cerebral infarction at an elevated accuracy and so on, as being able to be practically used.

### Means for Solving the Problems

As a result of extensive research to achieve the above-mentioned object, the present inventors found that the values which are obtained newly by mathematically statistically analyzing the contents of acrolein, and interleukin-6 and CRP which are inflammatory markers, in subject samples, polyamine oxidase activity and subjects' ages are significantly statistically correlated with whether or not to have asymptomatic cerebral infarction, and the invention was thus accomplished based on the results.

More specifically, the present invention includes each aspect as described below.

(1) A method of detecting cerebral stroke or asymptomatic cerebral infarction, comprising measuring the contents of acrolein polyamine, interleukin-6 and C-reactive protein, polyamine oxidase activity or a polyamine oxidase protein content in a biological sample obtained from a subject and using the measurement data thus obtained and a subject's age as indications.
(2) the use of a kit for detecting cerebral stroke or asymptomatic cerebral infarction by a method as claimed in claim 1, comprising a reagent for measuring the contents of acrolein interleukin-6 and C-reactive protein, polyamine oxidase activity or a polyamine oxidase protein content in a biological sample.
(3) the use of a system for detecting cerebral stroke or asymptomatic cerebral infarction by a method as claimed in claim 1, comprising: a reagent for measuring the contents of acrolein , interleukin-6 and C-reactive protein, polyamine oxidase activity or a polyamine oxidase protein content in a biological sample; and an electronic treating device and software for carrying out mathematically statistical analysis.

### Advantages of the Invention

The method according to an aspect of the present invention allows detection of a patient with cerebral stroke or an asymptomatic patient at a high accuracy of about 95%. Accordingly, a patient with cerebral stroke or asymptomatic cerebral infarction can be detected and diagnosed early before developing a symptom of cerebral infarction, or can be screened.

### Brief Description of the Drawings

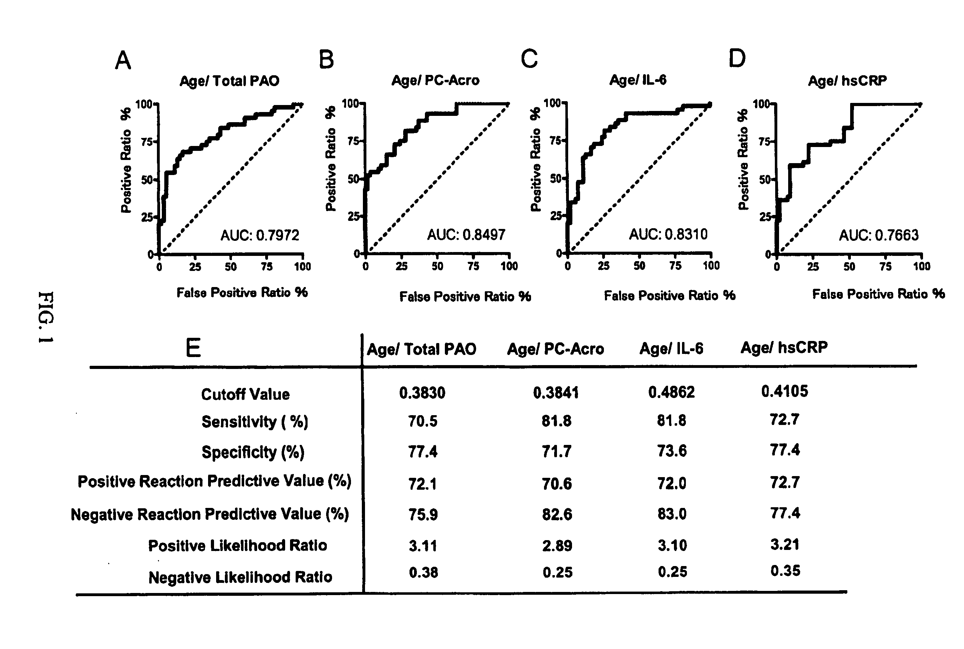

FIG. 1 shows each ROC curve, cutoff value, sensitivity, specificity, predictive value and likelihood ratio in a combination of subjects' ages with one selected from four plasma biomarkers (polyamine oxidase, acrolein, interleukin-6 and CRP) in a group of patients with asymptomatic cerebral infarction, and healthy subjects;

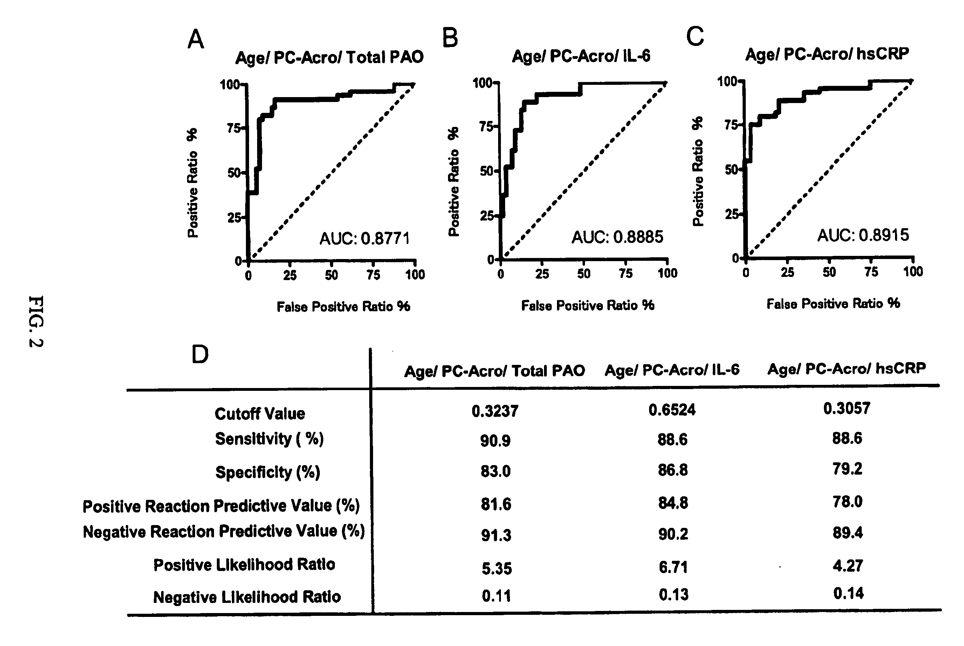

FIG. 2 shows each ROC curve, cutoff value, sensitivity, specificity, predictive value and likelihood ratio in a combination of the subjects' ages and the acrolein with one selected from the other three plasma biomarkers (the polyamine oxidase, the interleukin-6 and the CRP) in the group of the patients with asymptomatic cerebral infarction, and the healthy subjects;

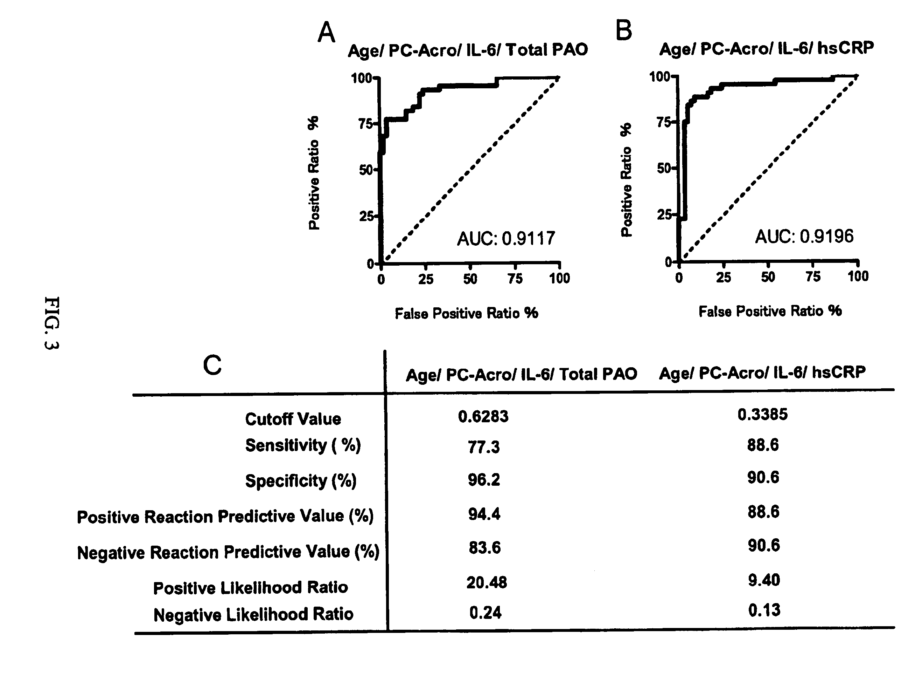

FIG. 3 shows each ROC curve, cutoff value, sensitivity, specificity, predictive value and likelihood ratio in a combination of the subjects' ages, the acrolein and the interleukin-6 with one selected from the other two plasma biomarkers (the polyamine oxidase and the CRP) in the group of the patients with asymptomatic cerebral infarction, and the healthy subjects; and

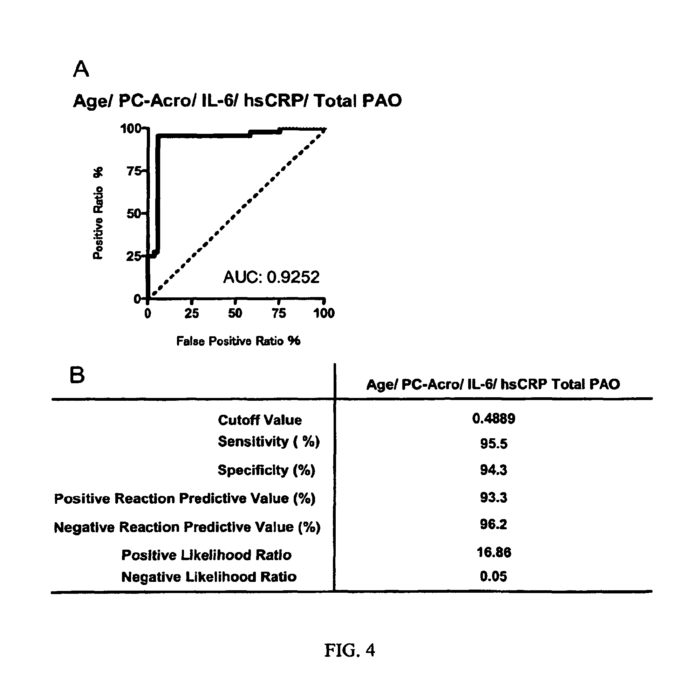

FIG. 4 shows an ROC curve, a cutoff value, a sensitivity, a specificity, a predictive value and a likelihood ratio using the subjects' ages, the acrolein, the polyamine oxidase, the interleukin-6 and the CRP in the group of the patients with asymptomatic cerebral infarction, and the healthy subjects.

### Best Mode for Carrying Out the Invention

A most preferred embodiment of the present invention will be described in detail below, but many other different embodiments according to the present invention, which are apparent to those skilled in the art, are possible, and the present invention is not limited to the embodiment described below.

The biological samples from the subjects, used in the method according to an aspect of the present invention, are preferably plasma used in an example as described below. However, other biological samples can also be used appropriately. Such other biological samples can include, for example, urine, saliva, cerebral spinal fluid and bone marrow fluid.

The term "polyamine" herein refers to a straight-chain aliphatic hydrocarbon having two or more primary amino groups. Known biogenic polyamines may include, but are not limited to, putrescine, cadaverine, spermidine, spermine, 1,3-diaminopropane, caldine, homospermidine, 3-aminopropylcadaverine, norspermine, thermospermine, caldopentamine, and so on. Preferred polyamines in accordance with an aspect of the present invention may be putrescine, spermidine and spermine.

The above polyamines are metabolized by oxidation, acetylation, transamination or carbamoylation, and a polyamine oxidase (acetylpolyamine oxidase (AcPAO) or spermine oxidase (SMO)) is an enzyme involved in the oxidation of polyamines. The term "polyamine oxidase" herein refers to an enzyme that oxidizes a diamine or a polyamine as a good substrate and generates hydrogen peroxide. Such a polyamine is oxidatively deaminated by a polyamine oxidase, resulting formation of aldehyde compounds such as acrolein.

A plasma acrolein content can be determined by any procedure well-known to those skilled in the art, for example, measuring the content of FDP-lysine (N-formyl-piperidino-lysine), which is an amino acid adder with acrolein. An FDP-lysine content can be measured, for example, by using ACR-LYSINE ADDUCT ELISA SYSTEM (NOF CORPORATION), according to the attached manual. The acrolein content may also be measured in the form of derivatives other than FDP-lysine. Furthermore, it is also possible to measure the acrolein content directly, and such a procedure is described in a report, for example, by Alarcon et al. (Alarcon, R. A. (1968) Anal. Chem. 40, 1704-1708). However, there is such a problem that the reactivity of acrolein with other molecules is high so that the amount of free acrolein in the blood is very small. Thus, considering the measurement of acrolein in the form of FDP-lysine is simple and easy, it is a preferred aspect in the present invention to measure acrolein in the form of FDP-lysine.

Specifically, subject plasma and a standard solution are dispensed into a plate immobilized with antigen by 50 µl/well, and further the same amount of primary antibody solution is added. The fluid is removed after left at rest for 30 minutes at room temperature and washed with a washing solution, followed by dispensing 100 µl/well of secondary antibody solution. It is washed with the washing solution after left at rest for 1 hour at room temperature, then color is developed by adding a coloring reagent of 100 µl/well and leaving it at rest for 15 minutes at room temperature, and reaction stop solutions are dispensed by 50 µl/well, followed by determining the absorbance at 450 nm using a plate reader. A plasma acrolein content is displayed as an FDP-lysine content per milliliter of plasma (nmo/ml plasma).

A plasma interleukin-6 content can be measured by any procedure well-known to those skilled in the art, for example, by using Human IL-6 ELISA (ENDOGEN CORPORATION), according to the attached manual.

Specifically, primary antibody solutions are dispensed into a 98-well plate by 50 µl/well, and further the same amounts of subject plasma and standard solution are added. The fluid is removed after left at rest for 2 hours at room temperature and washed with a washing solution, followed by dispensing 100 µl/well of secondary antibody solution. It is washed with the washing solution after left at rest for 30 minutes at room temperature, then color is developed by adding a coloring reagent of 100 µl/well and leaving it at rest for 30 minutes at room temperature, and reaction stop solutions are dispensed by 100 µl/well, followed by determining the absorbance at 450 nm using a plate reader. A plasma interleukin-6 content is displayed as a plasma content per milliliter of plasma (pg/ml plasma).

A plasma CRP content can be measured by any procedure well-known to those skilled in the art, for example, by using Human CRP ELISA KIT (Alpha Diagnostics International, Inc.), according to the attached manual.

Specifically, each well is washed with a washing solution, followed by dispensing subject plasma and standard solution into a 98-well plate by 10 µl/well and further adding an abzyme labeling solution of 100 µl/well. The fluid is removed after left at rest for 30 minutes at room temperature and washed with a washing solution. A coloring reagent is added, color is developed while shaking the mixture for 10 minutes at room temperature, and reaction stop solutions are dispensed by 50 µl/well, followed by determining the absorbance at 450 nm using a plate reader. A plasma CRP content is displayed as a CRP content per milliliter of patient plasma (mg/dl plasma).

The measurement of the activities of polyamine oxidases (AcPAO and SMO) can be conducted by any procedure well-known to those skilled in the art, for example, by incubating 0.06 ml of reaction mixture containing 10 mM Tris-hydrochloric acid (pH 7.5), 0.2 mM substrate (acetyl spermine or spermine) and 0.05 ml of patient plasma for 48 hours at 37°C. Trichloroacetic acid (TCA) is added to 0.02 ml of the reaction mixture to a final concentration of 5%, and it is centrifuged. A part of obtained supernatant is used for polyamine assay. Polyamine oxidase activity can be displayed as the content of a spermidine generated by the decomposition of acetyl spermine or spermine per milliliter of patient plasma (nmol/ml plasma).

The procedures of measuring the enzyme activity of polyamine oxidase are described in various reports, which may include a report by Sharmin et al. (Sharmin et al., (2001) Biochem. Biophys. Res. Commun. 282, 228-235), a report by Sakata et al. (Sakata et al., (2003) Biochem. Biophys Res. Commun. 305, 143-149), and a report by Igarashi et al. (Igarashi et al., (1986) J. Bacteriol. 166, 128-134). Based on the descriptions of such reports, those skilled in the art can measure the enzymatic activity of polyamine oxidase by making appropriate modifications.

Furthermore, the protein content of polyamine oxidase can be measured by any procedure well-known to those skilled in the art, for example, by enzyme-linked immunosorbent assay (ELISA), western blotting analysis or immunoprecipitation method using a specific antibody for polyamine oxidase. Such procedures are heretofore known and commonly used, and therefore, those skilled in the art can measure the protein content of the enzyme using the above procedures by setting adequate conditions appropriately. An antibody against polyamine oxidase used for conducting these measurements may be a monoclonal antibody or a polyclonal antibody.

The polyclonal antibody against polyamine oxidase can be obtained, for example, by immunizing rabbits with the peptide fragment of polyamine oxidase by using a conventional technique for production of a peptide antibody. The production of a peptide antibody can be confirmed through assaying whether the antibody has reached to sufficient titer by collecting blood from rabbits administered with the peptide and measuring its antibody titer. The procedures for producing a peptide antibody are described in various experimental manuals and well known to those skilled in the art, so the antibody against polyamine oxidase can be obtained by making various modifications based on those descriptions.

In accordance with an aspect of the present invention, the contents of acrolein , interleukin-6 and C-reactive protein, polyamine oxidase activity or a polyamine oxidase protein content in the biological sample are measured; mathematically statistical analysis of the measurement data thus obtained and a subject's age is carried out, thereby consequently obtaining a value which exhibits a statistically significant change; and cerebral stroke or asymptomatic cerebral infarction can be detected based on the value.

The mathematical statistical analysis may be carried out by using procedures well-known to those skilled in the art, preferably by using a neural network technique. The neural network technique can be conducted, for example, by using NEUROSIM/L (Fujitsu Ltd.), according to the attached manual.

"Cutoff value" is a value set for the detection of a specific disease. Asymptomatic cerebral infarction can be detected using a value obtained as described above as "cutoff value." Cerebral stroke or asymptomatic cerebral infarction can also be detected using the value obtained as described above, which statistically significantly varies as "cutoff value." For example, in a patient with asymptomatic cerebral infarction and a healthy subject, an ROC (Receiver Operating Characteristic) curve is developed by using commercially available statistical analysis software based on values obtained by measuring the contents of the above biomarkers, the optimal sensitivity and specificity are determined, and a cutoff value may be set depending on the purpose of detection, for example, so that a higher sensitivity is given priority in detection for a purpose such as primary screening and a specificity is higher in detection for the purpose of close investigation.

Based on the findings in accordance with an aspect of the present invention, a method of prophylaxis of cerebral stroke and prevention of progress of the symptom by suppressing the contents of acrolein, and interleukin-6 and CRP which are inflammatory markers, polyamine oxidase activity or these protein contents in a subject sample can also be provided.

Furthermore, there is also discloses a method of screening (exploration) of a new drug which is effective for treatment of cerebral stroke by administering an experimental animal with a candidate compound which may be effective for treatment of cerebral stroke and assaying whether the compound has the activity of inhibiting the contents of acrolein, and interleukin-6 and CRP which are inflammatory markers, polyamine oxidase activity or these protein contents in the experimental animal.

In accordance with an aspect of the present invention, the use of a kit for detecting cerebral stroke or asymptomatic cerebral infarction by a method as claimed in claim 1 is further provided. The kit , comprises a reagent for measuring the contents of acrolein formed from polyamine, interleukin-6 and C-reactive protein, polyamine oxidase activity or a polyamine oxidase protein content in a biological sample. Furthermore, e.g., any measuring instrument or apparatus, standard solution and buffer well-known to those skilled in the art may be optionally contained.

In accordance with an aspect of the present invention, there is also provided the use of system for detecting cerebral stroke or asymptomatic cerebral infarction by a method as claimed in claim 1 comprising: a reagent for measuring acrolein , interleukin-6 and C-reactive protein, polyamine oxidase activity or a polyamine oxidase protein content in a biological sample; and an electronic treating device and software for carrying out mathematically statistical analysis. Such electronic treating devices for carrying out mathematically statistical analysis may include, e.g., an appropriate computer well-known to those skilled in the art; and such software for carrying out mathematically statistical analysis may include, e.g., one allowing the above neural network technique.

### Example

Hereinafter, the present invention will be further concretely described referring to an example concerning the construction of a cerebral infarction judgment model employing an artificial neural network, but the invention is not limited thereto.

The presence or absence of infarction was examined by taking head tomographic images by magnetic resonance imaging (MRI) with the permission and consent of subjects. An as a result, the subject group was divided into 44 patients with asymptomatic cerebral infarction and 53 healthy subjects. Blood was collected from the patients with asymptomatic cerebral infarction and the healthy subjects to compare the contents of acrolein, interleukin-6 (IL-6) and CRP and polyamine oxidase activity in plasma.

The plasma acrolein content was determined by measuring the content of FDP-lysine (N-formyl-piperidino-lysine), which is an amino acid adder with acrolein. The above procedure was conducted using ACR-LYSINE ADDUCT ELISA SYSTEM (NOF CORPORATION). The plasma interleukin-6 content was measured by the above procedure using Human IL-6 ELISA (ENDOGEN CORPORATION). The plasma CRP content was measured by the above procedure using Human CRP ELISA KIT (Alpha Diagnostics International, Inc.). The measurement of the activities of polyamine oxidases (AcPAO and SMO) was conducted by the above procedure.

To construct a neural network model, NEUROSIM/L version 4 (Fujitsu) was used as software, and learning was conducted by an error back-propagation method at learning constants of a learning rate (e) = 5.0 and a momentum term (a) = 0.4. In the neural network, three input layers were set at five of plasma biomarkers (polyamine oxidase, acrolein, interleukin-6 and CRP) and a subject's age, and, based on empirical rules, intermediate and output layers were set at three and one, respectively. Values of "1" for the patients with asymptomatic cerebral infarction and "0" for the healthy subjects were input into teacher data in an output layer. Learning data were from 97 samples in total consisting of 44 samples from the patients with asymptomatic cerebral infarction and 53 samples from the healthy subj ects.

To judge the presence or absence of asymptomatic cerebral infarction by the practical detection of the images and to examine the accuracy of the results of prediction in the neural network model, receiver operating characteristic (hereinafter, ROC) curve analyses and areas under the ROC curve (AUC) were calculated using the predicted values calculated by the above software analysis. GraphPad PRISM 4(GraphPad Software Corporation) was used for the calculation of the ROC curve analyses and the AUCs. The accuracy of the cutoff values was evaluated with sensitivity, specificity, predictive values (positive and negative reaction predictive values) and likelihood ratios (positive and negative likelihood ratios).

FIG. 1 shows the results of the ROC curve analyses in the combinations of the subjects' ages with one selected from the four plasma biomarkers (polyamine oxidase, acrolein, interleukin-6 and CRP) in the input layer. The compared AUCs of acrolein, interleukin-6, polyamine oxidase and CRP were ranked in the descending order. The combination of the ages with the interleukin-6 showed that the sensitivity and the specificity for the prediction of asymptomatic cerebral infarction were 81.8% and 73.6%, respectively; and these obtained results were comparable to those in the case of acrolein.

FIG. 2 shows the results of the ROC curve analyses in the combinations of the subjects' ages and the acrolein with one selected from the other three plasma biomarkers (polyamine oxidase, interleukin-6 and CRP) in the input layer. The compared AUCs of CRP, interleukin-6 and polyamine oxidase were ranked in the descending order. The combination of the ages and the acrolein with the interleukin-6 showed that the sensitivity and the specificity for the prediction of asymptomatic cerebral infarction were 88.6% and 86.8%, respectively, exhibiting improvement in reaction predictive value compared to the results in FIG. 1.

FIG. 3 shows the results of the ROC curve analyses in the combinations of the subjects' ages, the acrolein and the interleukin-6 with one selected from the other two plasma biomarkers (polyamine oxidase and CRP) in the input layer. The combination of the ages, the acrolein and the interleukin-6 with CRP showed that the sensitivity and the specificity for the prediction of asymptomatic cerebral infarction were 88.6% and 90.6%, respectively, exhibiting improvement in reaction predictive value compared to the results in FIG. 2.

FIG. 4 shows the results of the ROC curve analyses in the combination of the subjects' ages, the acrolein, the interleukin-6, CRP and polyamine oxidase in the input layer. For the prediction of asymptomatic cerebral infarction, the sensitivity, the specificity, the positive reaction predictive value, the negative reaction predictive value, the positive likelihood ratio and the negative likelihood ratio were 95.5%, 94,3%, 93.3%, 96.2%, 16.86 and 0.05, respectively. The reaction predictive values and the likelihood ratios were best improved, compared to the results in FIGs.1-3. Based on the results, the use of the artificial neural network technique resulted in the improvement in the judgment accuracy of the presence or absence of asymptomatic cerebral infarction.

### Industrial Applicability

Cerebral stroke or asymptomatic cerebral infarction can be detected and diagnosed early by using the detection method according to an aspect of the present invention. Furthermore, a possibility of paving a new way for prophylaxis or early treatment of a cerebral stroke and asymptomatic cerebral infarction, such as development of a drug for prophylaxis or treatment of cerebral stroke and asymptomatic cerebral infarction, is provided by exploring a compound that removes acrolein, polyamine oxidase, and interleukin-6 and CRP which are inflammatory markers by utilizing the findings of the invention.

## Claims

1. A method of detecting asymptomatic cerebral infarction, comprising:
a) measuring the amounts of:
i) acrolein;
ii) interleukin-6
iii) C-reactive protein; and
iv) polyamine oxidase activity or a polyamine oxidase protein, in a biological sample obtained from a subject; and
b) using the measurement data thus obtained and the subject's age as indications.

2. Use of a kit for detecting asymptomatic cerebral infarction by a method as claimed in claim 1, said kit comprising reagents for measuring the amounts in a biological sample of:
i) acrolein;
ii) interleukin-6
iii) C-reactive protein; and
iv) polyamine oxidase activity or a polyamine oxidase protein.

3. Use of a system for detecting asymptomatic cerebral infarction by a method as claimed in claim 1, said system comprising:
a) reagents for measuring the amounts in a biological sample of:
i) acrolein;
ii) interleukin-6
iii) C-reactive protein; and
iv) polyamine oxidase activity or a polyamine oxidase protein; and
b) an electronic treating device and software for carrying out a mathematical statistical analysis.

## Patentansprüche

1. Verfahren zum Detektieren einer asymptomatischen zerebralen Infarktbildung, das umfasst:
a) Messen der Mengen von:
i) Acrolein,
ii) Interleukin-6
iii) C-reaktivem Protein; und
iv) Polyamin-Oxidase-Aktivität oder Polyamin-Oxidase-Protein
in einer biologischen Probe, die von einer Person erhalten wird; und
b) Verwenden der Messdaten, die auf diese weise erhalten werden, und des Alters der Person als Angaben.

2. Verwendung eines Ausrüstsatzes zum Detektieren einer asymptomatischen zerebralen Infarktbildung durch ein Verfahren nach Anspruch 1, wobei der Ausrüstsatz Reagenzien zum Messen der Mengen von:
i) Acrolein,
ii) Interleukin-6
iii) C-reaktivem Protein; und
iv) Polyamin-Oxidase-Aktivität oder Polyamin-Oxidase-Protein
in einer biologischen Probe enthält.

3. Verwendung eines Systems zum Detektieren einer asymptomatischen zerebralen Infarktbildung durch ein Verfahren nach Anspruch 1, wobei das System enthält:
a) Reagenzien zum Messen der Mengen von
i) Acrolein,
ii) Interleukin-6
iii) C-reaktivem Protein; und
iv) Polyamin-Oxidase-Aktivität oder Polyamin-Oxidase-Protein,
in einer biologischen Probe; und
b) eine elektronische Behandlungsvorrichtung und Software zum Ausführen einer mathematischen statistischen Analyse.

## Revendications

1. Procédé de détection d'un infarctus cérébral asymptomatique, comprenant :
a) de mesurer les quantités :
i) d'acroléine ;
ii) d'interleukine-6 ;
iii) de protéine C réactive ; et
iv) d'activité de polyamine oxydase ou d'une protéine de polyamine oxydase, dans un échantillon biologique obtenu à partir d'un sujet ; et
b) d'utiliser les données de mesure ainsi obtenues et l'âge du sujet en tant qu'indications.

2. Utilisation d'un kit pour détecter un infarctus cérébral asymptomatique par l'intermédiaire d'un procédé tel que revendiqué dans la revendication 1, ledit kit comportant des réactifs pour mesurer les quantités dans un échantillon biologique :
i) d'acroléine ;
ii) d'interleukine-6 ;
iii) de protéine C réactive ; et
iv) d'activité de polyamine oxydase ou d'une protéine de polyamine oxydase.

3. Utilisation d'un système pour détecter un infarctus cérébral asymptomatique par l'intermédiaire d'un procédé tel que revendiqué dans la revendication 1, ledit système comportant :
a) des réactifs pour mesurer les quantités dans un échantillon biologique :
i) d'acroléine ;
ii) d'interleukine-6 ;
iii) de protéine C réactive ; et
iv) d'activité de polyamine oxydase ou d'une protéine de polyamine oxydase ; et
b) un dispositif et un logiciel de traitement électronique pour effectuer une analyse statistique mathématique.
